# EUROPEAN PATENT SPECIFICATION

(11) **EP 2 497 515 B1**
(45) Date of publication and mention of the grant of the patent: **15.01.2014**
(21) Application number: 12158559.0
(22) Date of filing: 08.03.2012
(51) Int. Cl.: A61M 16/08, A61M 16/06

(54) **Respiratory system connector**
Anschluss für ein Beatmungssystem
Connecteur de système respiratoire

(30) Priority: 11.03.2011 GB 201104163
(43) Date of publication of application: 12.09.2012
(73) Proprietor: Intersurgical AG, Vaduz (LI)
(72) Inventor: Bowsher, Richard Francis, Reading, Berkshire RG31 7DB (GB)
(74) Representative: Adamson Jones

(56) References cited:
- EP-A1- 1 640 034
- GB-A- 2 090 639
- US-A- 5 975 077
- US-A1- 2003 200 970
- US-A1- 2004 244 804

## Description

The present invention relates to a connector for a respiratory system and, more particularly, although not exclusively, to a connector for connection in the flow path between a patient interface and a flow driver.

Respiratory flow or ventilation systems typically comprise one or more devices for controlling or regulating flow to a patient interface, such as a mask, and a series of ducts for providing a flow path to and from the patient interface. The nature of the devices and the flow connections formed there-between is dependent on the type or method of ventilation being undertaken.

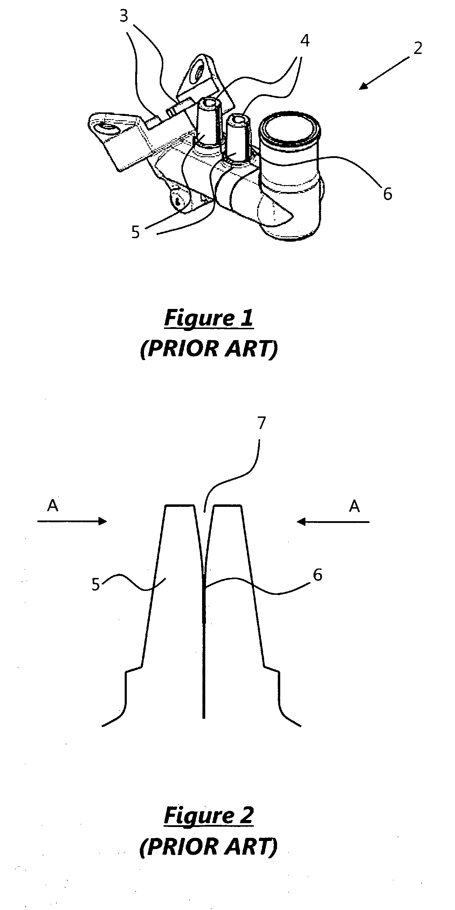

In Figure 1, there is shown a connector 2 for a continuous positive airway pressure (CPAP) system. The connector has a first set of ports 3 for connection to a patient mask and a second set of ports 4, which are in fluid communication with the first set of ports 3 via internal channels within the connector 2. In use, tube ends are pushed over the spigot formations 5 of the ports 4 to allow fluid communication with the mask via the tubes (not shown).

The complex internal geometry of the connector 2 dictates that the connector is manufactured as two individual components which are joined at their opposing faces along line 6. However the manufacture of the individual component parts by injection moulding has been found to cause a likelihood that the individual components can become warped in the vicinity of the spigot formation 5. Such warping may occur as the component cools, and, in particular, during ejection of the component part from a moulding tool.

Figure 2 shows an exaggerated example of a connection between the spigot formations 5 on opposing warped component parts. The deformation or incorrect formation of the component parts can lead to a discontinuity 7 in the join 6 between the component spigot halves.

Whilst such manufacturing imperfections do not prevent formation of the connector, it has been determined by the inventor that, upon connection of a tube, a compression force A is applied to the spigot by the tube, which causes internal stress within the spigot such that the spaced ends of the deformed spigot halves are pressed together. The compression force is necessary to provide a tight fit between the tube and spigot 5. This compression force has been found to be sufficient to cause a stress fracture, resulting in the possible failure of the connector in use.

Given that ventilation systems are used in life-critical scenarios, the correct functioning of such systems is of paramount importance. It is an aim of the present invention to provide a connector and associated respiratory system, for which the above problems are mitigated.

US 5,975,077 discloses a connector of the type discussed above. EP 1 640 034 discloses mask ports for attaching supplemental oxygen tubes or measurement devices to a respiratory mask, which are downwardly directed and recessed into the base of a mask frame. The ports may comprise a pair of downwardly extending tubular spigots, each housed in a respective recess in the base, with a shallow bridging recess there-between for receiving a bridging piece of a closure cap.

According to a first aspect of the present invention, there is provided a respiratory ventilation connector as recited in Claim 1.

The arrangement of an upstanding circumferential wall which can accommodate the tube therein avoids the need for a compression force in order to ensure a seal between the tube and connector and thus avoids the internal stress associated with the prior art, which can lead to failure of the device. A force resulting from the accommodation of a tube in the spigot formation is generally lower than in the prior art connector and is resolved by way of hoop stress rather than by compression of the spigot.

In one embodiment each of the first and second body portion comprise a partial port formation, such that the first port is defined by the combined partial port formations of the first and second body portions when connected.

The first and second body portions may comprise respective opposing surfaces in which the partial port formation and/or the partial passage formation are formed. Accordingly, the complex structure of connected internal passageways can be formed by moulding of the first and second body portions as separate members which can be connected together.

The spigot may take the form of a generally tubular upstanding wall formation disposed about the first port. The spigot may be spaced from the port by an end wall or stop formation. The first port may be formed in the end wall. The stop formation serves to correctly locate the tube end within the spigot at a predetermined depth such that the connection with the tube is not dependent on the insertion force.

The spigot may have a depth dimension which is approximately equal to its internal diameter. The ratio between the depth dimension and the internal diameter of the spigot may be between 1:2 and 2:1.

The spigot formation may comprise a first spigot formation.

The connector body may be shaped to define a further port, which may be adjacent said first port. Each body portion may comprise further respective spigot portions arranged in use to form an upstanding circumferential spigot formation about the further port, the further spigot formations having an internal diameter which is greater than the diameter of the further port so as to allow reception of a tubular fluid conduit within the further spigot for communication with the further port in use.

The first and further spigots may be located adjacent one another in a common surface of the connector body. The first and further spigots may share a common adjoining wall portion and may be connected thereby. A dual spigot arrangement of this kind serves to improve the structural rigidity of the first and further spigots during manufacture and/or use.

The upstanding circumferential spigot formations may be tapered in shape from a thicker wall section towards the connector body to a thinner wall section towards the open end of the spigot. An outer wall of the spigot formations may be substantially cylindrical or untapered in shape.

The further spigot may be formed in a manner similar to that of the first spigot by way of opposing spigot portions in each of the first and second connector body portions. The further port may communicate with a further internal passageway within the connector body. The construction features of either of the first port, spigot and/or internal passageway may also apply to the further port, spigot and/or internal passageway.

The connector body may comprise a third port arranged for communication with the first port via the internal passageway. The second port may be formed in the first body portion. The first body portion may be shaped to define an outer surface having an opening defining the second port. The third port may be formed in the second body portion. The second body portion may have an outer surface with an opening therein defining the third port.

The connector body may be shaped to provide an exhaust port, which may comprise a fourth port. The connector body may comprise a pair of exhaust ports arranged to communicate with a corresponding exhaust connector in use.

The first and second body portions may be joined at an intermediate plane at the interface between the opposing faces of the body portions. The first and/or further ports, and their corresponding spigot formations, may be substantially symmetrical about the intermediate plane. The second and/or third ports may lie to one side of the intermediate plane. The first passageway may have a junction therein such that the first port communicates with both the second and third ports. The second and third ports may communicate with the first port by respective passageways having a diameter which is smaller than the diameter of one or more exhaust passageways in the connector.

In one embodiment, the first and second body portions are fixed together by an adhesive. The first and second body portions may be formed of a plastic material which may be injection moulded to form the required shape.

The first and/or further ports may be arranged for connection to fluid conduits leading to one or more respiratory ventilation flow devices, such as, for example a flow driver or regulator device. The first port may receive fluid from a flow driver via a humidifier device, connected to the first port by the fluid conduit.

The first port may be an inlet port for receiving gas from a flow driver and the second port may lead to a patient interface.

According to the invention, there is provided a respiratory ventilation connector assembly comprising the connector and a first fluid conduit located within the spigot formation.

The first fluid conduit may comprise an end which is located in the spigot formation. The conduit end may be in abutment with an end wall or stop portion of the connector body located between the first port and spigot formation. The internal diameter of the fluid conduit may be substantially equal to, or less than, the diameter of the first port.

The assembly may comprise a further fluid conduit located in the further spigot formation in communication with the further port. The arrangement of the second or further fluid conduit in the further spigot formation may be the same as or similar to the arrangement of the first fluid conduit in the first spigot formation.

The assembly may comprise an exhaust connector connected to the connector at one or more exhaust ports.

The assembly may comprise a patient interface connected to the second and/or third ports. The second and/or third ports may comprise nasal flow ports.

The connector and/or assembly may comprise an infant flow connector, which may be suitable for neonatal respiratory use, such as, for example, within a CPAP system.

The term 'spigot' as used herein may be considered to comprise an adaptor for the coupling of fluidic devices or components to allow fluid flow there-between.

Practicable embodiments of the present invention are described in further detail below with reference to the accompanying drawings, of which:
Figure 1 shows a three-dimensional view of a respiratory system connector according to the prior art;
Figure 2 shows details of a spigot for a connector according to the prior art;
Figure 3 shows a schematic of a respiratory system in which the present invention may be used;
Figure 4 shows a three-dimensional view of a connector according to an embodiment of the present invention;
Figure 5 shows a plan view of the connector of Figure 4;
Figure 6 shows an exploded view of a connector according to an embodiment of the invention;
Figure 7 shows a first section view of the connector of Figure 5 at plane A-A with conduits connected for use; and,
Figure 8 shows a second section view of the connector of Figure 5 at plane B-B.

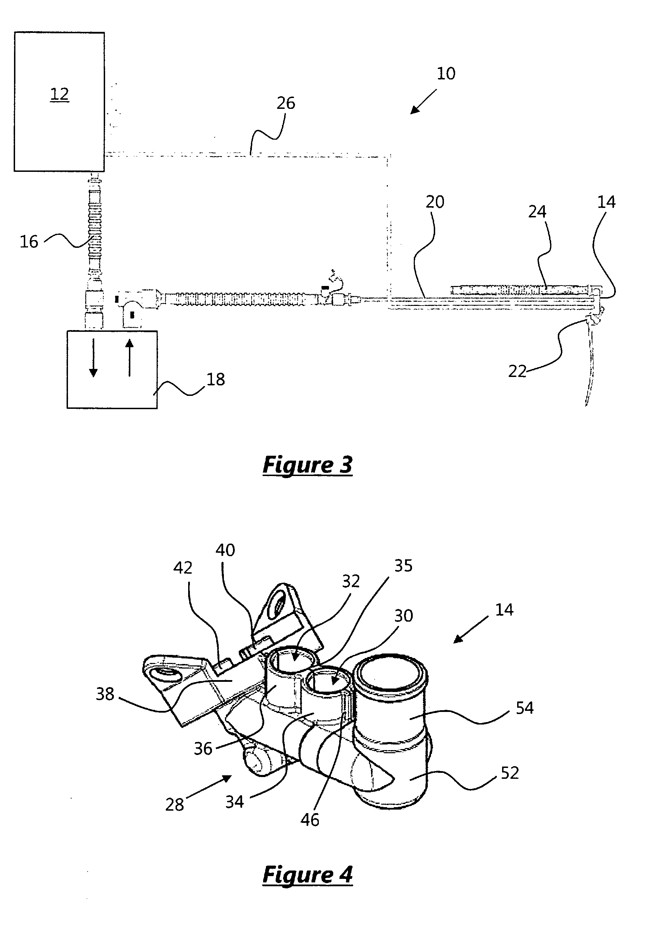

Turning firstly to Figure 3, there is shown a respiratory system in which a connector according to the present invention may be used. The system 10 may be referred to in the art as a continuous positive airway pressure (CPAP) system and comprises a flow driver 12 for pressurising airflow to the connector 14. The pressure provided by the flow driver 12 may be substantially constant in use. Flow from the flow driver 12 passes along conduit 16 to a humidifier 18 prior to passing to the connector 14 via conduit 20.

The flow passes through the connector 14 to a patient interface connection 22 for inhalation by a patient. Exhaled air returns through the connector 14 and passes through exhaust tube 24. The pressure at the patient interface connection 22 is also monitored by connection of conduit 26 to the patient interface via connector.

The particular system of Figure 3 allows for a CPAP delivery system for infants and accordingly the connector 14 provides for the splitting of the flow along 20 into two nasal flow ports for delivery to an infant. Accordingly the connector 14 comprises a complex geometry to accommodate all the above-described flow paths within a single device as will be described in further detail below. Whilst the invention is described with reference to a specific CPAP system, it will be appreciated that the connector may be applicable to other respiratory flow systems in which the flow demands require a complex internal passageway such that the connector is formed in two or more body portions and has spigot formations at the intersection between the body portions when assembled.

Turning now to Figures 4 to 6, there is shown a connector 14, comprising a main body 28 having ports 30 and 32 with corresponding spigot formations 34 and 36. The main body 28 is also shaped to provide a patient interface connector portion 38 with ports 40 and 42.

The main body is formed of two opposing body portions 28A and 28B, each of which is a reflection of the other. That is to say, the main body 28 is substantially symmetrical about a plane 44, at which the opposing body halves 28A and 28B are joined. The plane 44 bisects the spigot formations 34 and 36 of the connector. Accordingly each body portion 28A, 28B is shaped so as to provide half-spigot formations 34A, 36A and 34B, 36B respectively. The half-spigot formations are substantially hemi-cylindrical in form so as to form the generally cylindrical upstanding spigot formation 34, 36 which depend outwardly from the connector main body 28.

The spigot formations 34 and 36 are immediately adjacent such that they share a common adjoining wall portion 35. The spigot formations 34 and 36 also have a flange formation 46 aligned with the spigot, which is formed from opposing flange parts 46A and 46B in the respective body portions 28A and 28B. The mating flange faces of the body portions provide an enlarged mating surface between the body portions 28A and 28B in the vicinity of the spigots at the adjoining plane 44 and may also serve to improve the rigidity of the connector once formed. The flanges are substantially axial with reference to a spigot axis.

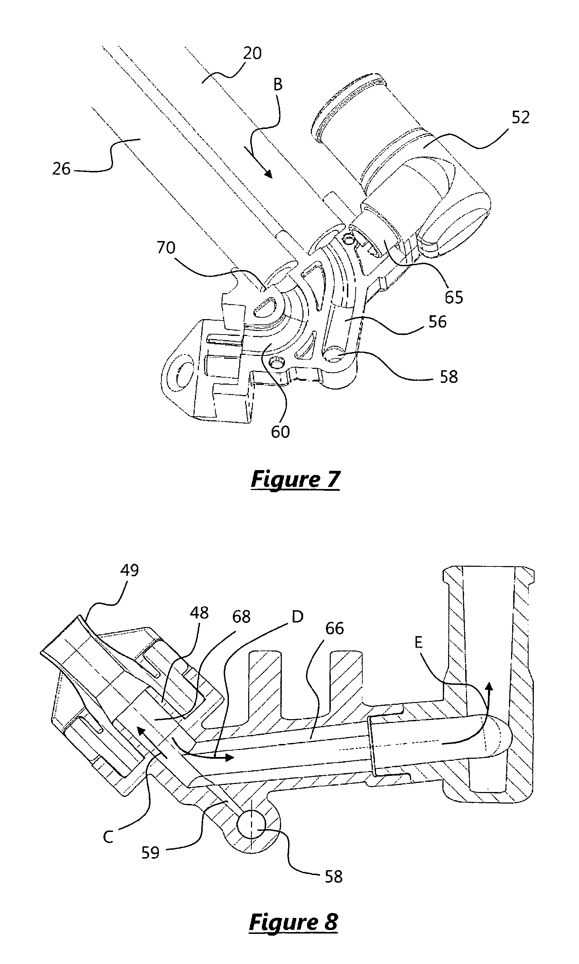

The ports 40 and 42 are provided with spigot formations in the form of upstanding male connectors 48 and 50, which are arrange to be connected in use to nasal adapters 49 - typically referred to as 'prongs' in the art - shown in Figure 8. However, nasal prongs are just one type of adapter or interface which may be attached and, in other embodiments, a different interface, such as a mask (not shown) may be used. A mask would typically be shaped to cover at least the nasal region of a patient so as to form a seal there-about in use.

The prongs 49 both feed the pressurised gas to the nostrils of a patient in use and also allow return flow during expiration. The patient interface connector portion 38 also has a base and side walls about its periphery, between which the connectors 48 and 50 are located. The nasal adapters and/or other type of interface is shaped to be closely seated within the side walls.

An exhaust duct 52 is fitted to the main body 28 and arranged to provide a passage for communication with the exhaust conduit 24 via connection formation 54 in use.

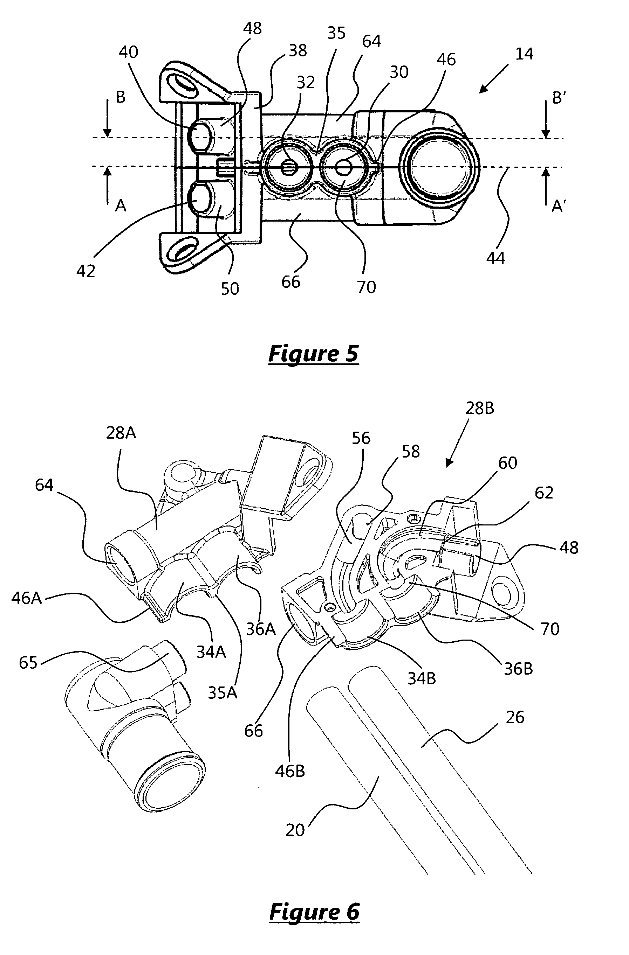

As can be seen in Figures 6 and 7, the body sections 28A and 28B have internal passageways formed therein. A first passageway 56 is formed by opposing grooves in the respective body portions 28A and 28B such that, when the body halves are brought together at plane 44, a complete internal passageway 56 is formed. Passage 56 leads from port 30 to a bifurcation or manifold 58 at which point the passage splits into separate passages which lead to respective ports 40 and 42.

Passage 60 is formed by the assembly of the body sections 28A and 28B in the manner described above in relation to passage 56. Passage 60 depends from port 32 and opens at its opposing end at a port 62 in the patient interface portion 38. Port 62 is located between spigots 48 and 50 in the patient interface portion 38 and is formed by opposing recesses in each body portion 28A and 28B.

Passage 60 allows for communication of fluid pressure between the patient interface and the airway pressure monitoring line 26. Flow in passage 60 is otherwise isolated from the flow in the other passages within the connector 14. That is to say, passage 60 does not communicate with the other internal passages within the connector 14. The remaining internal passages of connector 14, which allow for inspiration and expiration by the patient, are interconnected within the connector as will be described below.

In Figure 5, it can be seen that, in addition to passages 56 and 60 - which are formed at the plane 44 by opposing passage portion in each of the body sections 28A and 28B - there are also provided passages 64 and 66 which are spaced from the plane 44. Passages 64 and 66 are thus defined entirely within the body sections 28A and 28B respectively. Those passages communicate between the nasal ports 40, 42 and the exhaust duct 52. In this regard, passages 64 and 66 run along the outer sides of the connector and are spaced by the inner passages 56 and 60, which are formed there-between.

The exhaust duct 52 has a bifurcation therein such that the passages 64 and 66 are combined into a single exhaust flow away from connector 14. In this regard, the exhaust duct 54 has spaced protrusions 65 with male connector ends which locate in passages 64 and 66 to allow the exhaust duct to be attached to main body 28.

As can be seen in Figure 7, a common duct 68 depends from each of the ports 40 and 42, into which feed both the bifurcated duct 58 from passage 56 and also the exhaust passage 66.

Also visible in Figures 6 and 7 is the connection formed between the conduits 20 and 26 with the spigots 34 and 36 respectively. Conduits 20 and 26 are flexible tubes having blunt ends.

The ports 30 and 32 which form openings in the main body into passages 56 and 60 respectively are surrounded by the upstanding circumferential walls of the spigots 34 and 36. The internal diameter of the spigots 34 and 36 is larger than the size of the openings 30, 32 such that the openings are spaced from the corresponding spigot by an intermediate surface 70 portion which is annular in plan.

The diameter of each of ports 30 and 32 is equal to, or slightly smaller than, the internal diameter of respective conduits 20, 26. The tube ends are inserted into spigots 34 and 36. The intermediate surface 70 acts as an abutment for the tube 20, 26 ends. An adhesive is provided about the interface between the tube and the spigot formations, for example about the outer peripheral surface of the tube ends, to firmly hold the tubes 20, 26 in place.

The operation of the connector 14 will now be described once connected within the system shown in Figure 3 with reference to Figures 7 and 8.

During inspiration, a pressurised gas supply is provided from the flow driver 12 to the connector 14 via tube 20. Flow of gas into the connector during inspiration is in the direction of arrow B. The flow passes from tube 20 into the connector 14 at port 30 and along passage 56 to bifurcation 58, from where the flow splits as it passes into passages 68 and to a patient via ports 40 and 42 in the direction of arrow C. It is to be noted that the intermediate passages 59, which lead from the bifurcation 58 to passages 68 (see Figure 8), are of smaller width or diameter than that of passage 66.

During inspiration and expiration, static pressure at the patient interface (i.e. within the patient mask) is measured via passage 60.

During expiration, flow from the patient passes through nasal adapters 49 and ports 40 and 42 into passages 68. From passages 68, the exhaled gas passes in the direction of arrow D into passages 64 and 66. The exhaled breath does not return down intermediate passage 59 since passage 59 is pressurized in the opposite (inspiration) direction and also due to the reduced size of passage 59 compared to that of passages 64 or 66. Accordingly passage 59 is biased against exhaled air and instead the exhaled air passes into passages 64 and 66. During expiration, the gas supplied via tube 20 to intermediate passage 59 turns as it exits passage 59 with the prevailing expiration flow in the direction of arrow D. Accordingly the pressurised flow of gas from tube 20 assists the flow of exhaled gas towards the exhaust conduit along passages 64 and 66.

The combined flow from the patient and also from conduit 59 pass along passages 64 and 66 to the exhaust duct 52 and exits the connector 14 in the direction of arrow E via exhaust tube 24.

The inspiration and expiration cycle is then repeated.

The main body of the connector 14 is formed of a rigid plastic material which may be transparent. The mask and nasal adapters are made or a softer, resilient material, such as silicone. The conduits 20 and 26 are typically formed of a resilient polymer material.

## Claims

1. A respiratory connector assembly for connection in a flow path to a patient interface in a respiratory system, the connector assembly comprising:
a tubular fluid conduit (20;26) and
a connector (14) having first (28A) and second (28B) opposing body portions joined together so as to define a connector body (28) having a first port (30),
the connector body (28) further comprising a second port (32) and an internal passage (56;60) for fluid communication between said first (30) and second (32) ports,
each body portion (28A,28B) comprises respective first (34A) and second (34B) spigot portions arranged so as to form a spigot (34) about the first port (30), **characterised in that** the spigot (34) has an internal dimension which is greater than the size of the port (30), the tubular fluid conduit (20) being received within the spigot (34), and wherein adhesive is provided between an outer surface of the conduit (20) and an inner wall of the spigot (34).

2. A connector assembly as claimed in Claim 1, wherein each of the first (28A) and second (28B) body portion comprise a partial port formation, such that the first port (30) is defined by the combined partial port formations of the first (28A) and second (28B) body portions when connected.

3. A connector assembly as claimed in Claim 1 or 2, wherein each of the first (28A) and second (28B) body portion comprise a partial passage formation arranged such that the internal passage (56; 60) of the connector body (28) is defined by combined partial passage formations of the first (28A) and second (28B) body portions when connected.

4. A connector assembly as claimed in any preceding claim, wherein the first (28A) and second (28B) body portions are joined at respective opposing faces at a plane of contact (44) such that the spigot (34) intersects said plane.

5. A connector assembly as claimed in any preceding claim, wherein the spigot (34) comprises a generally annular or tubular wall which is upstanding from the connector body (28).

6. A connector assembly as claimed in any preceding claim, wherein the spigot (34) is spaced from the first port (30) by a stop formation (70).

7. A connector assembly as claimed in any preceding claim, wherein the spigot (34) has a depth dimension and an internal diameter, the ratio of which is between 1:2 and 2:1.

8. A connector assembly as claimed in any preceding claim, wherein the connector body (28) comprises a further spigot (36) arranged about a port (32), the first (28A) and second (28B) opposing body portions being joined together so as to define said port (32) and spigot (36) on the connector body (28).

9. A connector assembly as claimed in Claim 8, wherein the first (34) and further spigots (36) are immediately adjacent such that they share a common intermediate wall portion (35).

10. A connector assembly as claimed in any preceding claim, wherein the spigot (34;36) has a tapered interior wall surface.

11. A connector assembly as claimed in any preceding claim, wherein the connector body (28) comprises a third and fourth (40; 42) ports arranged for communication with the first port (30) via the internal passage, the internal passage comprising a bifurcation (58) such that the first port (30) is in fluid communication with both the third (40) and fourth (42) ports.

12. A connector assembly as claimed in Claim 11, wherein the third (40) and fourth (42) ports are formed in the first (28A) and second (28B) body portions respectively and are spaced from a join between the first (28A) and second (28B) body portion in the connector body (28).

13. A connector assembly as claimed in any preceding claim, further comprising an exhaust port and an exhaust passage (52) formed in the first (28A) or second (28B) body portion for fluid communication between the second port (32) and the exhaust port.

14. A respiratory connector assembly as claimed in any preceding claim, wherein an end of the fluid conduit (20; 26) abuts a stop formation (70) of the connector body (28) located between the first port (30) and spigot (34).

15. An infant and/or CPAP respiratory ventilation system comprising the connector assembly of any preceding claim, the first conduit being arranged for connection to a flow driver for providing flow to the first port of the connector, wherein the pressurised gas provided through the first port of the connector assists flow through the connector in both inspiration and expiration flow regimes.

## Patentansprüche

1. Verbinderbaugruppe für ein Beatmungsgerät zum Anschließen in einem Strömungsweg zu einer Patientenschnittstelle in einem Beatmungssystem, wobei die Verbinderbaugruppe Folgendes umfasst:
eine tubuläre Fluidleitung (20; 26) und
einen Verbinder (14) mit einem ersten (28A) und einem gegenüberliegenden zweiten (28B) Körperteil, die so aneinandergefügt sind, dass sie einen Verbinderkörper (28) mit einem ersten Anschluss (30) definieren,
wobei der Verbinderkörper (28) ferner einen zweiten Anschluss (32) und einen Innenkanal (56; 60) zur Fluidverbindung zwischen dem genannten ersten (30) und zweiten (32) Anschluss umfasst,
jeder Körperabschnitt (28A, 28B) jeweils einen ersten (34A) und zweiten (34B) Zapfenabschnitt umfasst, die so ausgelegt sind, dass sie einen Zapfen (34) um den ersten Anschluss (30) bilden, **dadurch gekennzeichnet, dass** der Zapfen (34) einen Innendurchmesser hat, der größer ist als der Anschluss (30), wobei die tubuläre Fluidleitung (20) in dem Zapfen (34) aufgenommen wird und wobei Klebstoff zwischen einer Außenfläche der Leitung (20) und einer Innenwand des Zapfens (34) vorgesehen ist.

2. Verbinderbaugruppe nach Anspruch 1, wobei der erste (28A) und der zweite (28B) Körperabschnitt jeweils eine Teilanschlussformation umfassen, so dass der erste Anschluss (30), wenn angeschlossen, von den kombinierten Teilanschlussformationen des ersten (28A) und zweiten (28B) Körperanschlusses definiert wird.

3. Verbinderbaugruppe nach Anspruch 1 oder 2, wobei der erste (28A) und zweite (28B) Körperabschnitt jeweils eine Teil-Kanalformation umfassen, die so angeordnet ist, dass der Innenkanal (56; 60) des Verbinderkörpers (28), wenn angeschlossen, durch kombinierte Teil-Kanalformationen des ersten (28A) und zweiten (28B) Körperabschnitts definiert wird.

4. Verbinderbaugruppe nach einem vorherigen Anspruch, wobei der erste (28A) und zweite (28B) Körperabschnitt an jeweiligen gegenüberliegenden Flächen in einer Kontaktebene (44) zusammengefügt werden, so dass der Zapfen (34) die genannte Ebene schneidet.

5. Verbinderbaugruppe nach einem vorherigen Anspruch, wobei der Zapfen (34) eine vom Verbinderkörper (28) abstehende allgemein ringförmige oder tubuläre Wand umfasst.

6. Verbinderbaugruppe nach einem vorherigen Anspruch, wobei der Zapfen (34) durch eine Anschlagformation (70) vom ersten Anschluss (30) beabstandet ist.

7. Verbinderbaugruppe nach einem vorherigen Anspruch, wobei der Zapfen (34) ein Tiefenmaß und einen Innendurchmesser hat, deren Verhältnis zwischen 1:2 und 2:1 liegt.

8. Verbinderbaugruppe nach einem vorherigen Anspruch, wobei der Verbinderkörper (28) einen weiteren Zapfen (36) umfasst, der um einen Anschluss (32) angeordnet ist, wobei der erste (28A) und zweite (28B) gegenüberliegende Körperabschnitt aneinander gefügt sind, um den genannten Anschluss (32) und den Zapfen (36) am Verbinderkörper (28) zu definieren.

9. Verbinderbaugruppe nach Anspruch 8, wobei der erste (34) und weitere Zapfen (36) unmittelbar benachbart sind, so dass sie einen gemeinsamen Zwischenwandabschnitt (35) haben.

10. Verbinderbaugruppe nach einem vorherigen Anspruch, wobei der Zapfen (34; 36) eine sich verjüngende Innenwandfläche hat.

11. Verbinderbaugruppe nach einem vorherigen Anspruch, wobei der Verbinderkörper (28) einen dritten und vierten (40; 42) Anschluss umfasst, die für eine Kommunikation mit dem ersten Anschluss (30) über den Innenkanal angeordnet sind, wobei der Innenkanal eine Verzweigung (58) aufweist, so dass der erste Anschluss (30) sowohl mit dem dritten (40) als auch dem vierten (42) Anschluss in Fluidverbindung ist.

12. Verbinderbaugruppe nach Anspruch 11, wobei der dritte (40) und vierte (42) Anschluss jeweils im ersten (28A) und zweiten (28B) Körperabschnitt ausgebildet und von einer Fügung zwischen dem ersten (28A) und zweiten (28B) Körperabschnitt im Verbinderkörper (28) beabstandet sind.

13. Verbinderbaugruppe nach einem vorherigen Anspruch, die ferner einen Auslassanschluss und einen Auslasskanal (52) umfasst, die im ersten (28A) oder zweiten (28B) Körperabschnitt für eine Fluidverbindung zwischen dem zweiten Anschluss (32) und dem Auslassanschluss ausgebildet sind.

14. Verbinderbaugruppe für ein Beatmungsgerät nach einem vorherigen Anspruch, wobei ein Ende der Fluidleitung (20; 26) an eine Anschlagformation (70) des Verbinderkörpers (28) anstößt, die sich zwischen dem ersten Anschluss (30) und dem Zapfen (34) befindet.

15. Kleinkind- und/oder CPAP-Beatmungsgerät-Ventilationssystem, das die Verbindergruppe nach einem vorherigen Anspruch umfasst, wobei die erste Leitung für eine Verbindung mit einem Strömungstreiber zum Erzeugen eines Flusses zum ersten Anschluss des Verbinders angeordnet ist, wobei das durch den ersten Anschluss des Verbinders bereitgestellte Druckgas beim Durchfluss durch den Verbinder bei Ein- und Ausatmungsflussvorgängen assistiert.

## Revendications

1. Ensemble connecteur respiratoire pour la connexion dans une voie d'écoulement vers une interface de patient dans un appareil respiratoire, l'ensemble connecteur comprenant :
un conduit de fluide tubulaire (20 ; 26) et
un connecteur (14) ayant des première (28A) et deuxième (28B) parties de corps opposées reliées entre elles de manière à définir un corps de connecteur (28) ayant un premier orifice (30),
le corps de connecteur (28) comprenant en outre un deuxième orifice (32) et un passage interne (56 ; 60) pour une communication fluide entre lesdits premier (30) et deuxième (32) orifices,
chaque partie de corps (28A, 28B) comprend des première (34A) et deuxième (34B) parties d'ergot respectives disposées de manière à former un ergot (34) autour du premier orifice (30), **caractérisé en ce que** l'ergot (34) a une dimension intérieure qui est supérieure à la taille de l'orifice (30), le conduit de fluide tubulaire (20) étant reçu au sein de l'ergot (34), et où un adhésif est prévu entre une surface externe du conduit (20) et une paroi interne de l'ergot (34).

2. Ensemble connecteur tel que revendiqué dans la Revendication 1, où chacune de la première (28A) et de la deuxième (28B) partie de corps comprend une formation d'orifices partielle, de telle sorte que le premier orifice (30) est défini par les formations d'orifice partielles combinées des première (28A) et deuxième (28B) parties de corps lorsqu'elles sont connectées.

3. Ensemble connecteur tel que revendiqué dans la Revendication 1 ou 2, où chacune de la première (28A) et de la deuxième (28B) partie de corps comprend une formation de passage partielle disposée de telle sorte que le passage interne (56 ; 60) du corps de connecteur (28) est défini par des formations de passage partielles combinées des première (28A) et deuxième (28B) parties de corps lorsqu'elles sont connectées.

4. Ensemble connecteur tel que revendiqué dans une quelconque revendication précédente, où les première (28A) et deuxième (28B) parties de corps sont jointes en correspondance de faces opposées respectives à un plan de contact (44) de telle sorte que l'ergot (34) coupe ledit plan.

5. Ensemble connecteur tel que revendiqué dans une quelconque revendication précédente, où l'ergot (34) comprend une paroi généralement annulaire ou tubulaire qui est verticale à partir du corps de connecteur (28).

6. Ensemble connecteur tel que revendiqué dans une quelconque revendication précédente, où l'ergot (34) est espacé du premier orifice (30) par une formation de butée (70).

7. Ensemble connecteur tel que revendiqué dans une quelconque revendication précédente, ou l'ergot (34) a une dimension en profondeur et un diamètre interne, dont le rapport se situe entre 1:2 et 2:1.

8. Ensemble connecteur tel que revendiqué dans une quelconque revendication précédente, où le corps de connecteur (28) comprend un ultérieur ergot (36) disposé autour d'un orifice (32), les première (28A) et deuxième (28B) parties de corps opposées étant reliées entre elles de manière à définir lesdits orifice (32) et ergot (36) sur le corps de connecteur (28).

9. Ensemble connecteur tel que revendiqué dans la Revendication 8, où les premier (34) et ultérieur (36) ergots sont immédiatement adjacents de telle sorte qu'ils partagent une partie de paroi intermédiaire commune (35).

10. Ensemble connecteur tel que revendiqué dans une quelconque revendication précédente, où l'ergot (34 ; 36) a une surface de paroi intérieure conique.

11. Ensemble connecteur tel que revendiqué dans une quelconque revendication précédente, où le corps de connecteur (28) comprend des troisième et quatrième (40 ; 42) orifices disposés pour la communication avec le premier orifice (30) par l'intermédiaire du passage interne, le passage interne comprenant une bifurcation (58) de telle sorte que le premier orifice (30) est en communication fluide avec les troisième (40) et quatrième (42) orifices.

12. Ensemble connecteur tel que revendiqué dans la Revendication 11, où les troisième (40) et quatrième (42) orifices sont formés dans les première (28A) et deuxième (28B) parties de corps, respectivement, et sont espacés d'une jointure entre la première (28A) et la deuxième (28B) partie de corps dans le corps de connecteur (28).

13. Ensemble connecteur tel que revendiqué dans une quelconque revendication précédente, comprenant en outre un orifice d'évacuation et un passage d'évacuation (52) formés dans la première (28A) ou la deuxième (28B) partie de corps pour une communication fluide entre le deuxième orifice (32) et l'orifice d'évacuation.

14. Ensemble connecteur respiratoire tel que revendiqué dans une quelconque revendication précédente, où une extrémité du conduit de fluide (20 ; 26) vient en butée contre une formation de butée (70) du corps de connecteur (28) située entre le premier orifice (30) et l'ergot (34).

15. Système de ventilation respiratoire pour nourrisson et/ou CPAP comprenant l'ensemble connecteur d'une quelconque des revendications précédentes, le premier conduit étant disposé pour la connexion à un conducteur d'écoulement pour fournir un écoulement vers le premier orifice du connecteur, où le gaz sous pression fourni à travers le premier orifice du connecteur facilite l'écoulement à travers le connecteur dans des régimes d'écoulement à la fois lors de l'inspiration et de l'expiration.
